# FASCICULE DE BREVET EUROPEEN

(11) **EP 1 009 365 B1**
(45) Date de publication et mention de la délivrance du brevet: **03.04.2002**
(21) Numéro de dépôt: 98933687.0
(22) Date de dépôt: 23.06.1998
(51) Int. Cl.: A61K 7/06, A61K 7/50

(54) **COMPOSITIONS LAVANTES ET CONDITIONNANTES A BASE DE SILICONE ET DE GOMME DE GALACTOMANNANE HYDROPHOBE**
HAUTPFLEGE- UND WASCHZUSAMMENSETZUNGEN DIE SILIKON UND EINEN HYDROPHOBEN GALAKTOMANNANGUMMI ENTHALTEN
WASHING AND CONDITIONING COMPOSITIONS BASED ON SILICON AND HYDROPHOBIC GUAR GUM

(30) Priorité: 02.07.1997 FR 9708346
(43) Date de publication de la demande: 21.06.2000
(73) Titulaire: L'OREAL, 75008 Paris (FR)
(72) Inventeur: DE LA METTRIE, Roland, F-78110 Le Vésinet (FR)
(74) Mandataire: Le Blainvaux Bellegarde, Françoise
(86) Numéro de dépôt international: FR9801315
(87) Numéro de publication internationale: WO9901105

(56) Documents cités:
- EP-A- 0 035 899
- EP-A- 0 281 360
- EP-A- 0 432 951
- US-A- 4 960 876
- US-A- 5 217 652

## Description

La présente invention est relative à des compositions de lavage et de conditionnement des matières kératiniques, en particulier des cheveux et/ou de la peau, à base de silicone, d'agent tensioactif et d'une gomme de galactomannane hydrophobe, ainsi qu'aux procédés de lavage et de conditionnement mettant en oeuvre ces compositions.

Les compositions de lavage des matières kératiniques, notamment les shampooings, sont bien connues dans l'état de la technique. On a déjà proposé dans le passé d'utiliser dans de telles compositions, des silicones (agents de conditionnement) afin de conférer aux matières traitées, notamment aux cheveux, de bonnes propriétés cosmétiques telles que la douceur, la brillance et la facilité de démêlage.

Compte tenu du caractère insoluble des silicones utilisables dans les compositions de lavage et de conditionnement, on cherche à maintenir les silicones en dispersion régulière dans le milieu sans cependant faire chuter la viscosité et les propriétés détergentes et moussantes des compositions. Les silicones doivent également être véhiculées sur les matières kératiniques traitées en vue de leur conférer, suivant l'application, des propriétés de douceur, de brillance et de démêlage.

Il existe à ce jour peu de moyens pour maintenir efficacement en suspension les silicones insolubles, car il s'agit là d'un problème difficile à résoudre ; à cet effet, on a déjà proposé l'utilisation des dérivés d'esters à longue chaîne (agents nacrants) ou des polysaccharides tels que la gomme de xanthane (gélifiants). Cependant, les agents nacrants présentent des problèmes de cristallisation qui entraînent une évolution (augmentation) de la viscosité des compositions au cours du temps ; les agents gélifiants présentent également des inconvénients, à savoir, d'une part que la mousse des compositions détergentes contenant de la gomme de xanthane se développe difficilement (mauvais démarrage de mousse) et que, d'autre part, les compositions n'ont pas une texture lisse et s'écoulent par paquets, ce qui est peu apprécié des utilisateurs.

EP-A-432951 décrit des compositions de shampooing comprenant des tensioactifs, un dérivé cationique de gomme de guar (chlorure de guar hydroxypropyl trimonium) et une silicone non volatile et insoluble. US5217652 décrit des compositions de shampooing comprenant des tensioactifs anioniques, un chlorure de guar hydroxypropyl trimonium et une diméthiconecopolyol.

La demanderesse a découvert, ce qui fait l'objet de l'invention, qu'en utilisant dans des compositions de lavage à base de silicones insolubles et d'agents tensioactifs, au moins une gomme de galactomannane hydrophobe, il était possible d'obtenir des compositions présentant une très bonne homogénéité et une stabilité améliorée, ainsi qu'une viscosité satisfaisante pour l'application sur les matières kératiniques.

Les compositions ainsi préparées possèdent en outre, de bonnes propriétés détergentes et moussantes et confèrent aux matières kératiniques, notamment aux cheveux et/ou à la peau, une grande douceur.

Ces compositions, lorsqu'elles sont appliquées sur les cheveux, possèdent, en plus de leurs propriétés lavantes, des propriétés de conditionnement des cheveux, c'est-à-dire que les cheveux traités sont lisses, se démêlent facilement, sont doux au toucher et la coiffure a du volume. Les cheveux ont un aspect naturel et non chargé.

Les compositions selon l'invention sont stables : en particulier, il ne se produit aucun relargage de la silicone ou d'épaississement incontrôlé de la composition au cours du temps. Les compositions présentent enfin une texture non filante et fondante. La mousse est aérée et se rince facilement.

L'invention a donc pour objet de nouvelles compositions de lavage et de conditionnement à base de silicone, d'agents tensioactifs et de gomme de galactomannane hydrophobe décrite ci-après.

Un autre objet de l'invention est constitué par le procédé de lavage et de conditionnement mettant en oeuvre de telles compositions.

L'invention a encore pour objet l'utilisation d'une gomme de galactomannane hydrophobe comme agent de mise en suspension d'une silicone dans une composition de lavage et de conditionnement contenant, dans un milieu aqueux cosmétiquement acceptable, des tensioactifs.

D'autres objets de l'invention apparaîtront à la lecture de la description et des exemples qui suivent.

Les compositions de lavage et de conditionnement des matières kératiniques, en particulier des cheveux et de la peau, conformes à l'invention comprennent, dans un milieu aqueux cosmétiquement acceptable, au moins une silicone, au moins un agent tensioactif possédant des propriétés détergentes et au moins d'une gomme de galactomannane hydrophobe.

Les galactomannanes sont des polysaccharides composés principalement d'unités galactose et mannose. La principale source de galactomannanes sont les endospermes de certaines graines de légumineuses telles que le guar, le caroube....
De préférence, les galactomannanes sont choisis parmi les gommes de guar.

Les gommes de galactomannane utilisables selon l'invention peuvent être solubles ou insolubles dans les compositions, mais de préférence elles sont insolubles.

Le terme "degré de substitution" signifie le nombre moyen de groupe hydroxyl substitués pour chaque unité anhydroglycosidique du galactomannane.

Le terme "substitution molaire" signifie le nombre moyen de moles de substituants pour chaque unité anhydroglycosidique du galactomannane.

Par gommes de galactomannane hydrophobes, on définit selon l'invention des gommes de galactomannane qui possèdent des substituants hydrophobes.

De préférence, ces gommes de galactomannane selon l'invention possèdent des substituants hydrophiles et des substituants hydrophobes.

Les galactomannanes selon l'invention ont de préférence une substitution molaire totale supérieure à 0,7 et comprennent de 0,7 à 4 substituants hydrophiles et de 0,0001 à 0,02 substituants hydrophobes par unité anhydroglycosidique. Le rapport molaire des substituants hydrophiles/ substituants hydrophobes est de préférence compris entre 35:1 et 40000:1.

Selon l'invention, les substituants hydrophiles peuvent être choisis parmi les groupements alkyles en C₁-C₄, les groupements hydroxyméthyl, hydroxyéthyl, hydroxypropyl, hydroxybutyl, carboxyméthyl et les groupements aminés et carboxyliques.

Selon l'invention, les substituants hydrophobes peuvent être choisis parmi les alkyles et les alkényles, linéaires ou ramifiés, ayant de 8 à 60 atomes de carbone et de préférence de 10 à 32 atomes de carbone et leurs mélanges, les groupements alkyles ou alkényles peuvent être substitués par un ou plusieurs hydroxyles.

Les substituants hydrophiles et hydrophobes peuvent être soit reliés directement par une liaison carbone-carbone à l'unité anydroglycosidique, soit par l'intermédiaire d'une liaison éther, uréthanne, ester, amide, acyle et de préférence par une liaison éther.

Plus particulièrement, les galactomannanes selon l'invention ont une substitution molaire totale allant de 0,9 à 2,01 et comprennent de 0,9 à 2 substituants hydrophiles et de 0,0005 à 0,01 substituants hydrophobes par unité anhydroglycosidique. Le rapport molaire des substituants hydrophiles/ substituants hydrophobes est compris entre 90:1 et 4000:1.

Plus particulièrement, le substituant hydrophile est le groupement hydroxypropyle et le substituant hydrophobes est un alkyle linéaire ayant de 16 à 28 atomes de carbone ou un mélange de tels alkyles, éventuellement relié aux unités anydroglycosidique par l'intermédiaire d'une liaison éther et comprenant un groupement hydroxyle.

Ces composés peuvent être préparés selon le procédé décrit dans les brevets US4,960,876 et US4,870,167.

Des gommes de galactomannane hydrophobe utilisables dans le cadre de la présente invention, sont notamment des gommes de guar commercialisées sous la dénomination ESAFLOR HM 22 par la société LAMBERTI ou sous la dénomination JAGUAR XC 95-3 par la société RHONE POULENC.

Les silicones utilisables conformément à l'invention sont en particulier des polyorganosiloxanes insolubles dans la composition et peuvent se présenter sous forme d'huiles, de cires, de résines ou de gommes.

Les organopolysiloxanes sont définis plus en détail dans l'ouvrage de Walter NOLL "Chemistry and Technoiogy of Silicones" (1968) Academie Press. Elles peuvent être volatiles ou non volatiles.

Lorsqu'elles sont volatiles, les silicones sont plus particulièrement choisies parmi celles possédant un point d'ébullition compris entre 60° C et 260° C, et plus particulièrement encore parmi :
(i) les silicones cycliques comportant de 3 à 7 atomes de silicium et de préférence 4 à 5. Il s'agit, par exemple, de l'octaméthylcyclotétrasiloxane commercialisé notamment sous le nom de "VOLATILE SILICONE 7207" par UNION CARBIDE ou "SILBIONE 70045 V 2" par RHONE POULENC, le décaméthylcyclopentasiloxane commercialisé sous le nom de 'VOLATILE SILICONE 7158" par UNION CARBIDE, "SILBIONE 70045 V 5" par RHONE POULENC, ainsi que leurs mélanges.
   On peut également citer les cyclocopolymères du type diméthylsiloxanes/ méthylakylsiloxane, tel que la "SILICONE VOLATILE FZ 3109" commercialisée par la société UNION CARBIDE, de structure chimique : 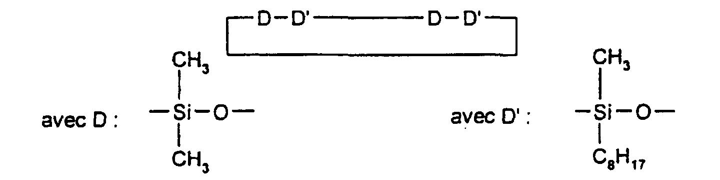
   On peut également citer les mélanges de silicones cycliques avec des composés organiques dérivés du silicium, tels que le mélange d'octaméthylcyclotétrasiloxane et de tétratriméthylsilylpentaérythritol (50/50) et le mélange d'octaméthylcyclotétrasiloxane et d'oxy-1,1'(hexa-2,2,2',2',3,3'-triméthylsilyloxy) bis-néopentane ;
(ii) les silicones volatiles linéaires ayant 2 à 9 atomes de silicium et possédant une viscosité inférieure ou égale à 5.10⁻⁶m²/s à 25° C. Il s'agit, par exemple, du décaméthyltétrasiloxane commercialisé notamment sous la dénomination "SH 200" par la société TORAY SILICONE. Des silicones entrant dans cette classe sont également décrites dans l'article publié dans Cosmetics and toiletries, Vol. 91, Jan. 76, P. 27-32 - TODD & BYERS "Volatile Silicone fluids for cosmetics".

On utilise de préférence des silicones non volatiles et plus particulièrement des polyalkylsiloxanes, des polyarylsiloxanes, des polyalkylarylsiloxanes, des gommes et des résines de silicones, des polyorganosiloxanes modifiés par des groupements organofonctionnels ainsi que leurs mélanges.

Ces silicones sont plus particulièrement choisies parmi les polyalkylsiloxanes parmi lesquels on peut citer principalement les polydiméthylsiloxanes à groupements terminaux triméthylsilyle ayant une viscosité de 5.10⁻⁶ à 2,5 m²/s à 25°C et de préférence 1.10⁻⁵ à 1 m²/s.

Parmi ces polyalkylsiloxanes, on peut citer à titre non limitatif les produits commerciaux suivants :
- les huiles SILBIONE des séries 47 et 70 047 ou les huiles MIRASIL commercialisées par RHONE POULENC telles que par exemple l'huile 70 047 V 500 000;
- les huiles de la série MIRASIL commercialisées par la société RHONE POULENC ;
- les huiles de la série 200 de la société DOW CORNING telles que plus particulièrement la DC200 de viscosité 60 000 Cst ;
- les huiles VISCASIL de GENERAL ELECTRIC et certaines huiles des séries SF (SF 96, SF 18) de GENERAL ELECTRIC.

On peut également citer les polydiméthylsiloxanes à groupements terminaux diméthylsilanol (Dimethiconol selon la dénomination CTFA) tels que les huiles de la série 48 de la société RHONE POULENC .

Dans cette classe de polyalkylsiloxanes, on peut également citer les produits commercialisés sous les dénominations "ABIL WAX 9800 et 9801" par la société GOLDSCHMIDT qui sont des polyalkyl (C₁-C₂₀) siloxanes.

Les polyalkylarylsiloxanes sont particulièrement choisis parmi les polydiméthyl méthylphénylsiloxanes, les polydiméthyl diphénylsiloxanes linéaires et/ou ramifiés de viscosité de 1.10⁻⁵ à 5.10⁻²m²/s à 25°C.

Parmi ces polyalkylarylsiloxanes on peut citer à titre d'exemple les produits commercialisés sous les dénominations suivantes :
. les huiles SILBIONE de la série 70 641 de RHONE POULENC ;
. les huiles des séries RHODORSIL 70 633 et 763 de RHONE POULENC ;
. l'huile DOW CORNING 556 COSMETIC GRAD FLUID de DOW CORNING ;
. les silicones de la série PK de BAYER comme le produit PK20 ;
. les silicones des séries PN, PH de BAYER comme les produits PN1000 et PH1000 ;
. certaines huiles des séries SF de GENERAL ELECTRIC telles que SF 1023, SF 1154, SF 1250, SF 1265.

Les gommes de silicone utilisables conformément à l'invention sont notamment des polydiorganosiloxanes ayant des masses moléculaires moyennes, en nombre élevées comprises entre 200 000 et 1 000 000 utilisés seuls ou en mélange dans un solvant. Ce solvant peut être choisi parmi les silicones volatiles, les huiles polydiméthylsiloxanes (PDMS), les huiles poly-phénylméthylsiloxanes (PPMS), les isoparaffines, les polyisobutylènes, le chlorure de méthylène, le pentane, le dodécane, le tridécanes ou leurs mélanges.

On peut plus particulièrement citer les produits suivants :
- polydiméthylsiloxane
- les gommes polydiméthylsiloxanes/méthylvinylsiloxane,
- polydiméthylsiloxane/diphénylméthylsiloxane,
- polydiméthylsiloxane/phénylméthylsiloxane,
- polydiméthylsiloxane/diphénylsiloxane/méthylvinylsiloxane.

Des produits plus particulièrement utilisables conformément à l'invention sont des mélanges tels que :
. les mélanges formés à partir d'un polydiméthylsiloxane hydroxylé en bout de chaîne (dénommé diméthiconol selon la nomenclature du dictionnaire CTFA) et d'un polydiméthylsiloxane cyclique (dénommé cyclométhicone selon la nomenclature du dictionnaire CTFA) tel que le produit Q2 1401 commercialisé par la société DOW CORNING ;
. les mélanges formés à partir d'une gomme polydiméthylsiloxane avec une silicone cyclique tel que le produit SF 1214 Silicone Fluid de la société GENERAL ELECTRIC, ce produit est une gomme SF 30 correspondant à une diméthicone, ayant un poids moléculaire moyen en nombre de 500 000 solubilisée dans l'huile SF 1202 Silicone Fluid correspondant au décaméthylcyclopentasiloxane ;
. les mélanges de deux PDMS de viscosités différentes, et plus particulièrement d'une gomme PDMS et d'une huile PDMS, tels que le produit SF 1236 de la société GENERAL ELECTRIC. Le produit SF 1236 est le mélange d'une gomme SE 30 définie ci-dessus ayant une viscosité de 20 m²/s et d'une huile SF 96 d'une viscosité de 5.10⁻⁶m²/s. Ce produit comporte de préférence 15 % de gomme SE 30 et 85 % d'une huile SF 96.

Les résines d'organopolysiloxanes utilisables conformément à l'invention sont des systèmes siloxaniques réticulés renfermant les unités :
R₂SiO_{2/2}, R₃SiO_{1/2}, RSiO_{3/2} et SiO_{4/2} dans lesquelles R représente un groupement hydrocarboné possédant 1 à 16 atomes de carbone ou un groupement phényle. Parmi ces produits, ceux particulièrement préférés sont ceux dans lesquels R désigne un radical alkyle inférieur en C₁-C₄, plus particulièrement méthyle, ou un radical phényle.

On peut citer parmi ces résines le produit commercialisé sous la dénomination "DOW CORNING 593" ou ceux commercialisés sous les dénominations "SILICONE FLUID SS 4230 et SS 4267" par la société GENERAL ELECTRIC et qui sont des silicones de structure diméthyl/triméthyl siloxane.

On peut également citer les résines du type triméthylsiloxysilicate commercialisées notamment sous les dénominations X22-4914, X21-5034 et X21-5037 par la société SHIN-ETSU.

Les silicones organo modifiées utilisables conformément à l'invention sont des silicones telles que définies précédemment et comportant dans leur structure un ou plusieurs groupements organofonctionnels fixés par l'intermédiaire d'un radical hydrocarboné.

Parmi les silicones organomodifiées, on peut citer les polyorganosiloxanes comportant :
- des groupements polyéthylèneoxy et/ou polypropylèneoxy comportant éventuellement des groupements alkyle en C₆-C₂₄ tels que les produits dénommés diméthicone copolyol commercialisé par la société DOW CORNING sous la dénomination DC 1248 ou les huiles SILWET L 722, L 7500, L 77, L 711 de la société UNION CARBIDE et l'alkyl (C₁₂) méthicone copolyol commercialisée par la société DOW CORNING sous la dénomination Q2 5200 ;
- des groupements aminés substitués ou non comme les produits commercialisés sous la dénomination GP 4 Silicone Fluid et GP 7100 par la société GENESEE ou les produits commercialisés sous les dénominations Q2 8220 et DOW CORNING 929 ou 939 par la société DOW CORNING. Les groupements aminés substitués sont en particulier des groupements aminoalkyle en C₁-C₄ ;
- des groupements thiols comme les produits commercialisés sous les dénominations "GP 72 A" et "GP 71" de GENESEE ;
- des groupements alcoxylés comme le produit commercialisé sous la dénomination "SILICONE COPOLYMER F-755" par SWS SILICONES et ABIL WAX 2428, 2434 et 2440 par la société GOLDSCHMIDT ;
- des groupements hydroxylés comme les polyorganosiloxanes à fonction hydroxyalkyle décrits dans la demande de brevet français FR-A-85: 16334 répondant à la formule (V) : 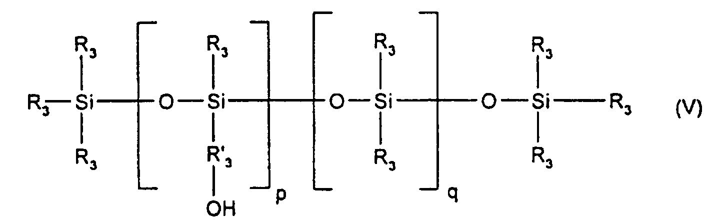 dans laquelle les radicaux R₃ identiques ou différents sont choisis parmi les radicaux méthyle et phényle ; au moins 60 % en mole des radicaux R₃ désignant méthyle ; le radical R'₃ est un chaînon alkylène divalent hydrocarboné en C₂-C₁₈ ; p est compris entre 1 et 30 inclus ; q est compris entre 1 et 150 inclus ;
- des groupements acyloxyalkyle tels que par exemple les polyorganosiloxanes décrits dans la demande de brevet français FR-A-2641185 et repondant à la formule (VI) : 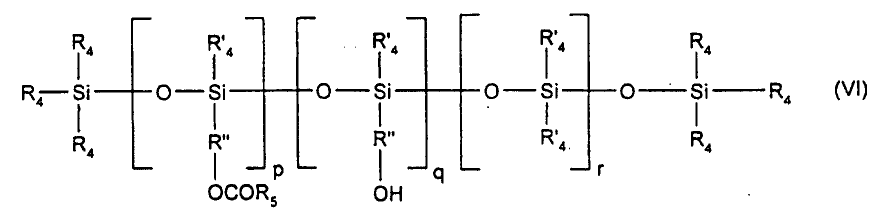
dans laquelle :
R₄ désigne un groupement méthyle, phényle, -OCOR₅, hydroxyle; un seul des radicaux
R₄ par atome de silicium pouvant être OH ;
R'₄ désigne méthyle, phényle ; au moins 60 % en proportion molaire de l'ensemble des radicaux R₄ et R'₄ désignant méthyle ;
R₅ désigne alkyle ou alcényle en C₈-C₂₀ ;
R" désigne un radical alkylène hydrocarboné divalent, linéaire ou ramifié en C₂-C₁₈ ;
r est compris entre 1 et 120 inclus ;
p est compris entre 1 et 30 ;
q est égal à 0 ou est inférieur à 0,5 p, p + q étant compris entre 1 et 30 ; les polyorganosiloxanes de formule (VI) peuvent contenir des groupements : 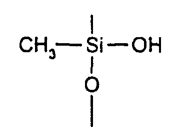 dans des proportions ne dépassant pas 15 % de la somme p + q +r.

Les composés de formule (VI) peuvent être préparés par estérification de polyorganosiloxanes à fonction hydroxyalkyle de formule (V) ci-dessus.
- des groupements anioniques du type carboxylique comme par exemple dans les produits décrits dans le brevet EP 186 507 de la société CHISSO CORPORATION, ou de type alkylcarboxyliques comme ceux présents dans le produit X-22-3701E de la société SHIN-ETSU ; 2-hydroxyalkylsulfonate ; 2-hydroxyalkylthiosulfate tels que les produits commercialisés par la société GOLDSCHMIDT sous les dénominations "ABIL S201" et "ABIL S255".
- des groupements hydroxyacylamino, comme les polyorganosiloxanes décrits dans la demande EP 342 834. On peut citer par exemple le produit Q2-8413 de la société DOW CORNING.

Selon l'invention, on peut également utiliser des silicones comprenant une portion polysiloxane et une portion constituée d'une chaîne organique non-siliconée, l'une des deux portions constituant la chaîne principale du polymère l'autre étant greffée sur la dite chaîne principale. Ces polymères sont par exemple décrits dans les demandes de brevet EP-A-412 704, EP-A-412 707; EP-A-640 105 et WO 95/00578, EP-A-582 152 et WO 93/23009 et les brevets US 4,693,935, US 4,728,571 et US 4,972,037. Ces polymères sont de préférence anioniques ou non ioniques.
De tels polymères sont par exemple les copolymères susceptibles d'être obtenus par polymérisation radicalaire à partir du mélange de monomères constitué par :
a) 50 à 90% en poids d'acrylate de tertiobutyle ;
b) 0 à 40% en poids d'acide acrylique ;
c) 5 à 40% en poids de macromère siliconé de formule : 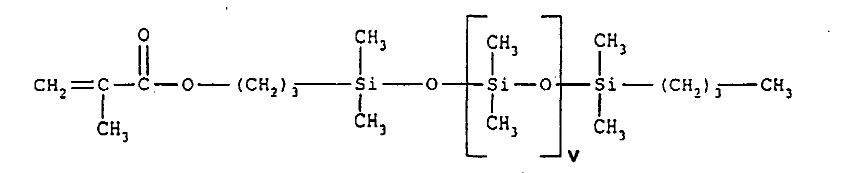 avec v étant un nombre allant de 5 à 700 ; les pourcentages en poids étant calculés par rapport au poids total des monomères.

D'autres exemples de polymères siliconés greffés sont notamment. des polydiméthylsiloxanes (PDMS) sur lesquels sont greffés, par l'intermédiaire d'un chaînon de raccordement de type thiopropylène, des motifs polymères mixtes du type acide poly(méth)acrylique et du type poly(méth)acrylate d'alkyle et des polydiméthylsiloxanes (PDMS) sur lesquels sont greffés, par l'intermédiaire d'un chaînon de raccordement de type thiopropylène, des motifs polymères du type poly(méth)acrylate d'isobutyle.

Selon l'invention, toutes les silicones peuvent également être utilisées sous forme d'émulsions.

Les polyorganosiloxanes particulièrement préférés conformément à l'invention sont :
- les silicones non volatiles choisies dans la famille des polyalkylsiloxanes à groupements terminaux triméthylsilyle telles que les huiles ayant une viscosité comprise entre 0,2 et 2,5 m²/s à 25° C telles que les huiles de la séries DC200 de DOW CORNING en particulier celle de viscosité 60 000 Cst, des séries SILBIONE 70047 et 47 et plus particulièrement l'huile 70 047 V 500 000 commercialisées par la société RHONE POULENC, les polyalkylsiloxanes à groupements terminaux diméthylsilanol tels que les diméthiconol ou les polyalkylarylsiloxanes tels que l'huile SILBIONE 70641 V 200 commercialisée par la société RHONE POULENC ;
- les mélanges d'organopolysiloxanes et de silicones cycliques tels que le produit Q2 1401 commercialisé par la société DOW CORNING, et le produit SF 1214 commercialisé par la société GENERAL ELECTRIC ;
- les mélanges de deux PDMS de viscosités différentes notamment d'une gomme et d'une huile tels que le produit SF 1236 commercialisé par la société GENERAL ELECTRIC ;
- la résine d'organopolysiloxane commercialisée sous la dénomination DOW CORNING 593 ;
- les polysiloxane à groupements aminés tels que les amodiméthicone ou les triméthylsilylamodiméthicone ;

Les agents tensioactifs utilisables dans les compositions de lavage et de conditionnement conformes à l'invention peuvent être choisis parmi les agents tensioactifs anioniques, amphotères, zwittérioniques, non ioniques ou leurs mélanges ayant des propriétés détergentes.

Leur nature ne revêt pas, dans le cadre de la présente invention, de caractère véritablement critique.
Les tensioactifs convenant à la mise en oeuvre de la présente invention sont notamment les suivants :

### (i) Tensioactif(s) anionique(s) :

Ainsi, à titre d'exemple de tensioactifs anioniques utilisables, seuls ou mélanges, dans le cadre de la présente invention, on peut citer notamment (liste non limitative) les sels (en particulier sels alcalins, notamment de sodium, sels d'ammonium, sels d'amines, sels d'aminoalcools ou sels de magnésium) des composés suivants : les alkylsulfates, les alkyléthersuffates; alkylamidoéthersulfates, alkylarylpolyéthersulfates, mono-glycérides sulfates ; les alkylsulfonates, alkylphosphates, alkylamidosulfonates, alkyl-arylsulfonates, α-oléfine-sulfonates, paraffine-sulfonates ; les alkylsulfosuccinates, les alkyléthersulfosuccinates, les alkylamidosulfosuccinates; les alkylsulfosuccinamates ; les alkylsulfoacétates ; les alkylétherphosphates; les acylsarcosinates ; les acyl-iséthionates et les N-acyltaurates, le radical alkyle ou acyle de tous ces différents composés comportant de préférence de 12 à 20 atomes de carbone, et le radical aryl désignant de préférence un groupement phényle ou benzyle. Parmi les tensioactifs anioniques encore utilisables, on peut également citer les sels d'acides gras tels que les sels des acides oléique, ricinoléique, palmitique, stéarique, les acides d'huile de coprah ou d'huile de coprah hydrogénée ; les acyl-lactylates dont le radical acyle comporte 8 à 20 atomes de carbone. On peut également utiliser les alkylpolyglycosides comportant un groupement sulfate, sulfonate, succinate ou sulfosuccinate, les acides d'alkyl D galactoside uroniques et leurs sels ainsi que les acides alkyl (C₆-C₂₄) éther carboxyliques polyoxyalkylénés, les acides alkyl(C₆-C₂₄)aryl éther carboxyliques polyoxyalkylénés ,les acides alkyl(C₆-C₂₄) amido éther carboxyliques polyoxyalkylénés et leurs sels, en particulier ceux comportant de 2 à 50 groupements oxyde d'éthylène, et leurs mélanges.

Parmi les tensioactifs anioniques, on préfère utiliser selon l'invention les sels d'alkylsulfates et d'alkyléthersufates et leurs mélanges.

### (ii) Tensioactif(s) non ionique(s) :

Les agents tensioactifs non-ioniques sont, eux aussi, des composés bien connus en soi (voir notamment à cet égard "Handbook of Surfactants" par M.R. PORTER, éditions Blackie & Son (Glasgow and London), 1991, pp 116-178) et leur nature ne revêt pas, dans le cadre de la présente invention, de caractère critique. Ainsi, ils peuvent être notamment choisis parmi (liste non limitative) les alcools, les alpha-diols, les alkylphénols ou les acides gras polyéthoxylés, polypropoxylés ou polyglycérolés, ayant une chaîne grasse comportant par exemple 8 à 18 atomes de carbone, le nombre de groupements oxyde d'éthylène ou oxyde de propylène pouvant aller notamment de 2 à 50 et le nombre de groupements glycérol pouvant aller notamment de 2 à 30. On peut également citer les copolymères d'oxyde d'éthylène et de propylène, les condensats d'oxyde d'éthylène et de propylène sur des alcools gras ; les amides gras polyéthoxylés ayant de préférence de 2 à 30 moles d'oxyde d'éthylène, les amides gras polyglycérolés comportant en moyenne 1 à 5 groupements glycérol et en particulier 1,5 à 4 ; les amines grasses polyéthoxylées ayant de préférence 2 à 30 moles d'oxyde d'éthylène ; les esters d'acides gras du sorbitan oxyéthylénés ayant de 2 à 30 moles d'oxyde d'éthylène ; les esters d'acides gras du sucrose, les esters d'acides gras du polyéthylèneglycol, les alkyl(C₈-C₂₀)polyglycosides éventuellement oxyalkylénés, les dérivés de N-alkyl glucamine, les oxydes d'amines tels que les oxydes d'alkyl (C₁₀-C₁₄) amines ou les oxydes de N-acylaminopropylmorpholine. On notera que les alkylpolyglycosides constituent des tensioactifs non-ioniques rentrant particulièrement bien dans le cadre de la présente invention.

### (iii) Tensioactif(s) amphotère(s):

Les agents tensioactifs amphotères, dont la nature ne revêt pas dans le cadre de la présente invention de caractère critique, peuvent être notamment (liste non limitative) des dérivés d'amines secondaires ou tertiaires aliphatiques, dans lesquels le radical aliphatique est une chaîne linéaire ou ramifiée comportant 8 à 22 atomes de carbone et contenant au moins un groupe anionique hydrosolubilisant (par exemple carboxylate, sulfonate, sulfate, phosphate ou phosphonate) ; on peut citer encore les alkyl (C₈-C₂₀) bétaïnes, les sulfobétaïnes, les alkyl (C₈-C₂₀) amidoalkyl (C₁-C₆) bétaïnes ou les alkyl (C₈-C₂₀) amidoalkyl (C₁-C₆) sulfobétaïnes.

Parmi les dérivés d'amines, on peut citer les produits commercialisés sous les dénomination MIRANOL, tels que décrits dans les brevets US-2 528 378 et US-2 781 354 et de structures :

R₂-CONHCH₂CH₂-N(R₃)(R₄)(CH₂COO-) (2)

dans laquelle : R₂ désigne un radical alkyle dérivé d'un acide R₂-COOH présent dans l'huile de coprah hydrolysée, un radical heptyle, nonyle ou undécyle, R₃ désigne un groupement bêta-hydroxyéthyle et R₄ un groupement carboxyméthyle ;
et

R₂-CONHCH₂CH₂-N(B)(C) (3)

dans laquelle :
B représente -CH₂CH₂OX', C représente -(CH₂)_{z} -Y', avec z = 1 ou 2,
X' désigne le groupement -CH₂CH₂-COOH ou un atome d'hydrogène
Y' désigne -COOH ou le radical -CH₂ - CHOH - SO3H
R_{2'} désigne un radical alkyle d'un acide R₉ -COOH présent dans l'huile de coprah ou dans l'huile de lin hydrolysée, un radical alkyle, notamment en C₇, C₉, C₁₁ ou C₁₃, un radical alkyle en C₁₇ et sa forme iso, un radical C₁₇ insaturé.

Ces composés sont classés dans le dictionnaire CTFA, Sème édition, 1993, sous les dénominations Disodium Cocoamphodiacetate, Disodium Lauroamphodiacetate, Disodium Caprylamphodiacetate, Disodium Capryloamphodiacetate, Disodium Cocoamphodipropionate, Disodium Lauroamphodipropionate, Disodium Caprylamphodipropionate, Disodium Capryloamphodipropionate, Lauroamphodipropionic acid, Cocoamphodipropionic acid.

A titre d'exemple on peut citer le cocoamphodiacetate commercialisé sous la dénomination commerciale MIRANOL C2M concentré par la société RHONE POULENC.

Dans les compositions conformes à l'invention, on utilise de préférence des d'agents tensioactifs anioniques et en particulier des mélanges d'agents tensioactifs anioniques et d'agents tensioactifs amphotères ou non ioniques. Un mélange particulièrement préféré est un mélange constitué d'au moins un agent tensioactif anionique et d'au moins un agent tensioactif amphotère.

On utilise de préférence un agent tensioactif anionique choisi parmi les alkyl(C₁₂-C₁₄) sulfates de sodium, de triéthanolamine ou d'ammonium, les alkyl (C₁₂-C₁₄)éthersulfates de sodium oxyéthylénés à 2,2 moles d'oxyde d'éthylène, le cocoyl iséthionate de sodium et l'alphaoléfine(C₁₄-C₁₆) sulfonate de sodium et leurs mélange avec :
- soit un agent tensioactif amphotère tel que les dérivés d'amine dénommés disodiumcocoamphodipropionate ou sodiumcocoamphopropionate commercialisés notamment par la société RHONE POULENC sous la dénomination commerciale "MIRANOL C2M CONC" en solution aqueuse à environ 38 % de matière active ou sous la dénomination MIRANOL C32;
- soit un agent tensioactif amphotère de type zwittérionique tel que les alkylbétaïnes en particulier la cocoylbétaïne commercialisée sous la dénomination "DEHYTON AB 30" en solution aqueuse à environ 32 % de MA par la société HENKEL.

La ou les gommes de galactomannane hydrophobes utilisées conformément à l'invention sont présentes de préférence dans des proportions comprises entre 0,1 et 10 % par rapport au poids total de la composition et en particulier entre 0,2 et 5 %.

La ou les silicones peuvent être utilisées dans les compositions conformes à l'invention dans des proportions généralement comprises entre 0,05 et 20 %, et de préférence entre 0,1 et 10 % en poids par rapport au poids total de la composition.

Le ou les agents tensioactifs sont généralement utilisés dans les compositions conformes à l'invention dans des proportions suffisantes pour conférer un caractère détergent à la composition, lesquelles sont comprises de préférence entre 5 et 50 % par rapport au poids total de la composition et en particulier entre 8 et 35 %.

Le véhicule, ou support, des compositions détergentes selon l'invention est de préférence de l'eau ou une solution hydroalcoolique d'un alcool inférieur en C₁-C₆ tel que éthanol, isopropanol ou butanol ou un mélange d'eau et d'alkylèneglycol comme le propylèneglycol et les éthers de glycol.

Les compositions détergentes selon l'invention présentent un pH final généralement compris entre 3 et 10. De préférence, ce pH est compris entre 5,5 et 8. L'ajustement du pH à la valeur désirée peut se faire classiquement par ajout d'une base (organique ou minérale) dans la composition, par exemple de l'ammoniaque, la soude ou une (poly)amine primaire, secondaire ou tertiaire comme la monoéthanolamine, la diéthanolamine, la triéthanolamine, l'isopropanolamine ou la propanediamine-1,3, ou encore par ajout d'un acide, de préférence un acide carboxylique tel que par exemple l'acide citrique.

Les compositions conformes à l'invention peuvent contenir en plus de l'association définie ci-dessus des agents régulateurs de viscosité tels que des électrolytes, ou des agents épaississants. On peut citer en particulier le chlorure de sodium, le xylène sulfonate de sodium, les scléroglucanes, les gommes de xanthane, les alcanolamides d'acide gras, les alcanolamides d'acide alkyl éther carboxylique éventuellement oxyéthylénés avec jusqu'à 5 moles d'oxyde d'éthylène tel que le produit vendu sous la dénomination "AMINOL A15" par la société CHEM Y, les acides polyacryliques réticulés et les copolymères acide acrylique / acrylates d'alkyle en C₁₀-C₃₀ réticulés. Ces agents régulateurs de viscosité sont utilisés dans les compositions selon l'invention dans des proportions pouvant aller jusqu'à 10 % en poids par rapport au poids total de la composition.

Les compositions conformes à l'invention peuvent également contenir jusqu'à 5 % d'agents nacrants ou opacifiants bien connus dans l'état de la technique tels que par exemple les palmitates de sodium ou de magnésium, les stéarates et hydroxystéarates de sodium ou de magnésium, les dérivés acylés à chaîne grasse tels que les monostéarates ou distéarates d'éthylène glycol ou de polyéthylèneglycol, les éthers à chaînes grasses telles que le disléaryléther ou le 1-hexadécyloxy octadodécanol.

Les compositions conformes à l'invention peuvent éventuellement contenir en outre d'autres agents ayant pour effet d'améliorer les propriétés cosmétiques de cheveux ou de la peau. On peut citer à ce sujet les agents tensioactifs cationiques, des polymères anioniques ou non ioniques ou cationiques ou amphotères, les protéines, les hydrolysats de protéines, les céramides, les pseudocéramides, les hydroxyacides, les vitamines, le panthénol, les huiles végétales, animales, minérales ou synthétiques, les agents antipelliculaires, les agents antisebborhéïques, les filtres solaires hydro ou liposolubles.

Parmi les agents tensioactifs cationiques on peut citer en particulier (liste non limitative) : les sels d'amines grasses primaires, secondaires ou tertiaires, éventuellement polyoxyalkylénées ; les sels d'ammonium quaternaire tels que les chlorures ou les bromures de tétraalkylammonium, d'alkylamidoalkyltrialkylammonium, de dialkyldihydroxyalkyl ammonium, de trialkylbenzylammonium, de trialkylhydroxyalkylammonium ou d'alkylpyridinium; les dérivés d'imidazoline, les diesters gras de diméthyl trihydroxyéthyl ammonium ; ou les oxydes d'amines à caractère cationique, les radicaux alkyles ayant de 1 à 4 atomes de carbone.

Les agents conditionneurs de type polymères cationiques utilisables conformément à la présente invention peuvent être choisis parmi tous ceux déjà connus en soi comme améliorant les propriétés cosmétiques des cheveux traités par des compositions détergentes, à savoir notamment ceux décrits dans la demande de brevet EP-A-0 337 354 et dans les demandes de brevets français FR-A-2 270 846, 2 383 660, 2 598 611, 2 470 596 et 2 519 863.

De manière encore plus générale, au sens de la présente invention, l'expression "polymère cationique" désigne tout polymère contenant des groupements cationiques et/ou des groupements ionisables en groupements cationiques.
Parmi tous les polymères cationiques susceptibles d'être utilisés dans le cadre de la présente invention, on préfère mettre en oeuvre les dérivés d'éther de cellulose quaternaires tels que les produits commercialisés sous la dénomination « JR 400 » par la société UNION CARBIDE CORPORATION, les cyclopolymères, en particulier les homopolymères de sel de diallyidiméthylammonium et les copolymères de sel de diallyldiméthylammonium et d'acrylamide en particulier les chlorures, commercialisés sous les dénominations « MERQUAT 100 », « MERQUAT 550 » et « MERQUAT S » par la société MERCK, les polysaccharides cationiques et plus particulièrement les gommes de guar modifiées par du chlorure de 2,3-époxypropyl triméthylammonium commercialisées par exemple sous la dénomination « JAGUAR C13S » par la société MEYHALL.

A titre de polymère amphotère, on peut citer les polymères comprenant au moins des unités diallyldiméthyl- ou diallyldiéthyl-ammonium et des unités acide acrylique tels que les produits commercialisés sous la dénomination MERQUAT 280 ou MERQUAT 295 par la société MERCK ;
- les chitosanes partiellement modifiés par des diacides carboxyliques en C₄-C₈ tels que ceux décrits dans le FR 2 137 684. Le taux de modification peut être compris entre 30 et 90% en poids par rapport au poids total du chitosane. Ces chitosanes peuvent être totalement désacétylés.

Selon l'invention, le ou les polymères cationiques peuvent représenter de 0,001 % à 10 % en poids, de préférence de 0,005 % à 5 % en poids, et encore plus préférentiellement de 0,01 % à 3 % en poids, du poids total de la composition finale.

Les compositions selon l'invention peuvent contenir également des synergistes de mousses tels que des 1,2-alcanediols en C₁₀-C₁₈ ou des alcanolamides gras dérivés de mono ou de diéthanoiamine.

Ces compositions peuvent également contenir différents adjuvants utilisés couramment en cosmétique tels que des parfums, des conservateurs, des agents séquestrants, des stabilisateurs de mousse bien connus en cosmétique.

Bien entendu, l'homme du métier veillera à choisir ce ou ces éventuels composés complémentaires et/ou leurs quantités de manière telle que les propriétés avantageuses attachées intrinsèquement à l'association (base lavante + silicone + gomme de guar) conforme à l'invention ne soient pas, ou substantiellement pas, altérées par la ou les adjonctions envisagées.

Ces compositions peuvent se présenter sous la forme de liquides plus ou moins épaissis, de crèmes ou de gel et elles conviennent principalement au lavage, au soin des matières kératiniques telles que la peau ou les cheveux.

Les compositions selon l'invention sont utilisés de préférence comme shampooings pour le lavage et le conditionnement des cheveux et ils sont appliqués dans ce cas-là sur le cheveux humides dans des quantités efficaces pour les laver, cette application étant suivie d'un rinçage à l'eau.

Les compositions conformes à l'invention sont également utilisables comme gels douche pour le lavage et le conditionnement des cheveux et de la peau, auquel cas ils sont appliqués sur la peau et les cheveux humides et sont rincés après application.

Les compositions conformes à l'invention sont également utilisables comme démaquillants des matières kératiniques telles que la peau, les cils, les sourcils.

L'invention a également pour objet un procédé de lavage et de conditionnement des matières kératiniques telles que les cheveux consistant à appliquer sur lesdites matières mouillées une quantité efficace d'une composition telle que définie ci-dessus, puis à effectuer un rinçage à l'eau après un éventuel temps de pause.

Des exemples concrets, mais nullement limitatifs, illustrant l'invention vont maintenant être donnés.

### EXEMPLE 1

On a réalisé une composition de shampooing contenant :
- Lauryléthersulfate de sodium (C12/C14 à 70/30) à 2,2 moles d'oxyde d'éthylène
   (MA = matière active) 16 gMA
- Tensioactif amphotère dérivé d'imidazoline commercialisé sous la dénomination MIRANOL C2M par la société RHONE POULENC à 40% de MA 3,2 gMA
- Gomme de guar hydroxypropylée modifiée par du stéaryl glycidyl éther commercialisée sous la dénomination JAGUAR XC 95-3 par la société RHONE POULENC 0,9 g
- Polydiméthylsiloxane commercialisé sous la dénomination de MIRASIL DM 500 000 par la société RHONE POULENC 2 g
- Acide citrique qs pH 5,5
- Parfum, conservateurs qs
- Eau déminéralisée qsp 100 g

La composition est stable et présente une bonne rhéologie.
On effectue un shampooing en appliquant environ 12 g de la composition sur des cheveux préalablement mouillés. On fait mousser le shampooing puis on rince abondamment à l'eau.
La mousse est aérée, elle s'élimine facilement.

Les cheveux se peignent facilement, présentent un toucher doux, et la chevelure a un du volume.

### EXEMPLE 2

On a réalisé une composition de shampooing contenant :
- Lauryléthersulfate de sodium (C12/C14 à 70/30) à 2,2 moles d'oxyde d'éthylène (MA = matière active) 14 gMA
- Tensioactif amphotère dérivé d'imidazoline commercialisé sous la dénomination MIRANOL C2M par la société RHONE POULENC à 40% de MA 2,8 gMA
- Polydiméthylsiloxane de viscosité 60 000 cSt commercialisé par la société DOW CORNING sous la dénomination FLUID DC 200 - 60 000 cSt 1 g
- Gomme de guar hydroxypropylée modifiée par du stéaryl glycidyl éther commercialisée sous la dénomination JAGUAR XC 95-3 par la société RHONE POULENC 0,75 g
- Hydroxyéthyl cellulose réticulé par de l'épichlorhydrine, et quaternisée par de la triméthylamine commercialisée sous la dénomination CELQUAT SC 240 par la société NATIONAL STARCH 0,2 g
- Alcool laurique oxyéthyléné à 2,5 moles d'oxyde d'éthylène 1,1 g
- Acide citrique qs pH 5,5
- Parfum, conservateurs qs
- Eau déminéralisée qsp 100 g

La composition est stable et présente une bonne rhéologie.
On effectue un shampooing en appliquant environ 12 g de la composition sur des cheveux préalablement mouillés. On fait mousser le shampooing puis on rince abondamment à l'eau.
La mousse est aérée, elle s'élimine facilement.

Les cheveux se peignent facilement, présentent un toucher doux, et la chevelure a du volume.

## Revendications

1. Composition de lavage et de conditionnement des matières kératiniques, en particulier des cheveux et de la peau, **caractérisée par le fait qu'**elle comprend, dans un milieu aqueux cosmétiquement acceptable, au moins une silicone, au moins un agent tensioactif possédant des propriétés détergentes et au moins une gomme de galactomannane qui possède des substituants hydrophobes, les substituants hydrophobes étant choisis parmi les alkyles et les alkényles, linéaires ou ramifiés, ayant de 8 à 60 atomes de carbone et de préférence de 10 à 32 atomes de carbone et leurs mélanges, les groupements alkyles ou alkényles peuvent être substitués par un ou plusieurs hydroxyles.

2. Composition selon la revendication 1, **caractérisée par le fait que** la -gomme de galactomannane possède des substituants hydrophiles et des substituants hydrophobes.

3. Composition selon la revendication 1 ou 2, **caractérisée par le fait que** la gomme de galactomannane présente une substitution molaire totale supérieure à 0,7.

4. Composition selon la revendication 2 ou 3, **caractérisée par le fait que** la gomme de galactomannane comprend de 0,7 à 4 substituants hydrophiles et de 0,0001 à 0,02 substituants hydrophobes par unité anhydroglycosidique.

5. Composition selon l'une quelconque des revendications 2 à 4,; **caractérisée par le fait que** le rapport molaire des substituants hydrophiles/ substituants hydrophobes est compris entre 35:1 et 40 000:1.

6. Composition selon l'une quelconque des revendications 2 à 5, **caractérisée par le fait que** les substituants hydrophiles sont choisis parmi les groupements alkyles en C₁-C₄, les groupements hydroxyméthyl, hydroxyéthyl, hydroxypropyl, hydroxybutyl, carboxyméthyl et les groupements aminés et carboxyliques.

7. Composition selon l'une quelconque des revendications 1 à 6, **caractérisée par le fait que** la gomme de galactomannane est une gomme de guar.

8. Composition selon l'une quelconque des revendications 1 à 7, **caractérisée par le fait que** les silicones sont choisies parmi les polyorganosiloxanes insolubles dans la composition et se présentent sous forme d'huiles, de cires, de résines ou de gommes.

9. Composition selon l'une quelconque des revendications 1 à 8, **caractérisée par le fait que** les polyorganosiloxanes sont des polyorganosiloxanes non volatils choisis parmi les polyalkylsiloxanes, les polyarylsiloxanes, les polyalkylarylsiloxanes, les gommes et résines de silicones, les polyorganosiloxanes modifiés par des groupements organofonctionnels ainsi que leurs mélanges.

10. Composition selon l'une quelconque des revendications 1 à 9, **caractérisée par le fait que** :
(a) les polyalkylsiloxanes sont choisis parmi :
- les polydiméthylsiloxanes à groupements terminaux triméthylsilyle ;
- les polydiméthylsiloxanes à groupements terminaux diméthylsilanol ;
- les polyalkyl(C₁-C₂₀)siloxanes ;
(b) les polyalkylarylsiloxanes sont choisis parmi :
- les polydiméthylméthylphénylsiloxanes, les polydiméthyldiphényl siloxanes linéaires et/ou ramifiés de viscosité comprise entre 1.10⁻⁶ et 5.10⁻²m²/s à 25°C ;
(c) les gommes de silicone sont choisies parmi les polydiorganosiloxanes ayant des masses moléculaires moyenne en poids comprises entre 200 000 et 1 000 000 utilisés seuls ou sous forme de mélange dans un solvant ;
(d) les résines sont choisies parmi les résines constituées d'unités : R₃ Si O_{1/2}, R₂ Si O_{2/2}, R Si O_{3/2}, Si O_{4/2}
dans lesquelles R représente un groupement hydrocarboné ayant de 1 à 16 atomes de carbone ou un groupement phényle ;
(e) les silicones organo modifiées sont choisies parmi les silicones comportant dans leur structure un ou plusieurs groupements organofonctionnels fixés par l'intermédiaire d'un radical hydrocarboné.

11. Composition selon la revendication 10, **caractérisée par le fait que** les gommes de silicone utilisées seules ou sous forme de mélange sont choisies parmi les structures suivantes :
- polydiméthylsiloxane
- polydiméthylsiloxane/méthylvinylsiloxanes,
- polydiméthylsiloxane/diphénysiloxane,
- polydiméthylsiloxane/phénylméthylsiloxane,
- polydiméthylsiloxane/diphénylsiloxane/méthylvinylsiloxanes et les mélanges suivants :
- des mélanges formés à partir d'un polydiméthylsiloxane hydroxylé en bout de chaîne et d'un polydiméthylsiloxane cyclique ;
- les mélanges formés à partir d'une gomme polydiméthylsiloxane et d'une silicone cyclique ; et
- des mélanges de polydiméthylsiloxanes de viscosités différentes.

12. Composition selon la revendication 10, **caractérisée par le, fait que** les silicones organomodifiées sont choisies parmi les polyorganosiloxanes comportant :
a) des groupements polyéthylèneoxy et/ou polypropylèneoxy ;
b) des groupements aminés substitués ou non ;
c) des groupements thiols ;
d) des groupements alcoxylés ;
e) des groupements hydroxyalkyle répondant à la formule suivante : 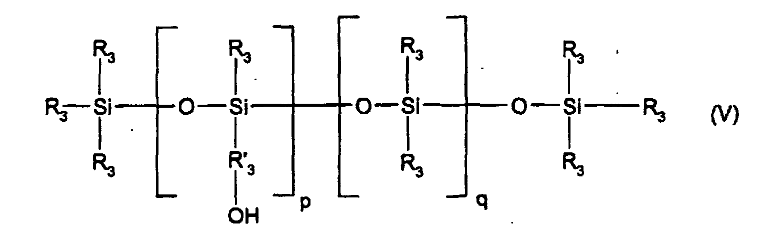 dans laquelle les radicaux R₃ identiques ou différents sont choisis parmi les radicaux méthyle et phényle, au moins 60 % en mole des radicaux R₃ représentant méthyle ; le radical R'₃ est un chaînon alkylène divalent hydrocarboné en C₂-C₁₈ ; p est compris entre 1 et 30 inclus ; q est compris entre 1 et 150 inclus ;
f) des groupements acyloxyalkyle répondant à la formule suivante : 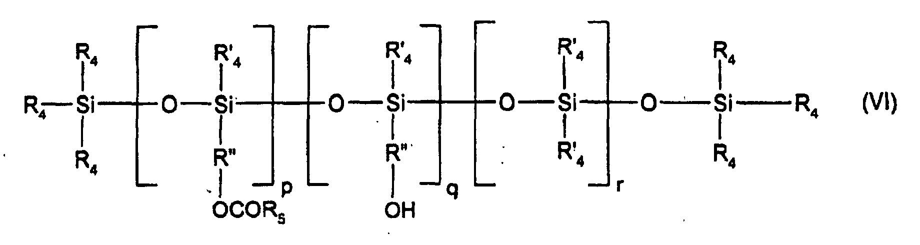 dans laquelle :
R₄ désigne méthyle, phényle, -OCOR₅, hydroxyle, un seul des radicaux R₄ par atome de silicium pouvant être OH ;
R'₄ désigne méthyle, phényle ; au moins 60 % en mole de l'ensemble des radicaux R₄ et
R'₄ désignant méthyle ;
R₅ désigne alkyle ou alcényle en C₈-C₂₀ ;
R" désigne un radical alkylène hydrocarboné divalent, linéaire ou ramifié en C₂-C₁₈ ;
r est compris entre 1 et 120 inclus ;
p est compris entre 1 et 30 ;
q est égal à 0 ou est inférieur à 0,5 p, p + q étant compris entre 1 et 30 ; les polyorganosiloxanes de formule (VI) pouvant contenir des groupements ; 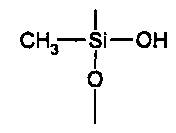
dans des proportions ne dépassant pas 15 % de la somme p + q + r ;
g) des groupements alkyl carboxyliques,
h) des groupements 2-hydroxyalkylsulfonates,
i) des groupements 2-hydroxyalkylthiosulfonates,
j) des groupements hydroxyacylamino.

13. Composition selon l'une quelconque des revendications 1 à 12, **caractérisée par le fait que** les polyorganosiloxanes sont choisis parmi les polyalkylsiloxanes à groupements terminaux triméthylsilyle, les polyalkylsiloxanes à groupements terminaux diméthylsilanol, les polyalkylarylsiloxanes, les mélanges de deux PDMS constitués d'une gomme et d'une huile de viscosités différentes, les mélanges d'organosiloxanes et de silicones cycliques, les résines d'organopolysiloxanes, les polysiloxanes à groupements aminés.

14. Composition selon l'une quelconque des revendications 1 à 8, **caractérisée par le fait que** les silicones sont choisies parmi les silicones volatiles.

15. Composition selon l'une quelconque des revendications 1 à 14, **caractérisée par le fait que** les agents tensioactifs détergents sont choisis parmi les agents tensioactifs anioniques, amphotères, non ioniques ou leurs mélanges.

16. Composition selon l'une quelconque des revendications 1 à 15, **caractérisée par le fait que** l'on utilise des mélanges de tensioactifs choisis parmi les mélanges de tensioactifs anioniques et d'agents tensioactifs amphotères ou non ioniques.

17. Composition selon l'une quelconque des revendications 1 à 16, **caractérisée par le fait que** la ou les silicones sont utilisées dans les compositions conformes à l'invention dans des proportions comprises entre 0,05 et 20 % et de préférence entre 0,1 et 10 % en poids par rapport au poids total de la composition.

18. Composition selon l'une quelconque des revendications 1 à 17, **caractérisée par le fait que** les gommes de galactomannane hydrophobes sont présentes dans des proportions comprises entre 0,1 et 10 % en poids par rapport au poids total de la composition et en particulier entre 0,2 et 5 %.

19. Composition selon l'une quelconque des revendications 1 à 18, **caractérisée par le fait que** les agents tensioactifs sont présents dans une proportion suffisante pour conférer un caractère détergent à la composition.

20. Composition selon la revendication 19, **caractérisée par le fait que** les agents tensioactifs sont présents dans des proportions comprises entre 5 et 50 % en poids par rapport au poids total de la composition en particulier entre 8 et 35 % en poids.

21. Composition selon l'une quelconque des revendications 1 à 20, **caractérisée par le fait que** le milieu aqueux est constitué par de l'eau ou par un mélange d'eau et d'un solvant cosmétiquement acceptable choisi parmi les alcools inférieurs, les alkylèneglycols et les éthers de glycol.

22. Composition selon l'une quelconque des revendications 1 à 21, **caractérisée par le fait qu'**elle comprend en plus un polymère cationique.

23. Utilisation comme shampooing de la composition telle que définie dans l'une quelconque des revendications 1 à 22.

24. Utilisation comme gel douche de la composition telle que définie dans l'une quelconque des revendications 1 à 22.

25. Procédé de lavage et de conditionnement des matières kératiniques, **caractérisé par le fait que** l'on applique sur ces matières au moins une composition telle que définie dans l'une quelconque des revendications 1 à 22, et qu'après un éventuel temps de pose on rince à l'eau les matières traitées.

26. Utilisation d'une gomme de galactomannane hydrophobe telle que définie à l'une des revendications 1 à 7, comme agent de mise en suspension d'une silicone dans une composition de lavage et conditionnement contenant, dans un milieu aqueux cosmétiquement acceptable, des tensioactifs.

## Patentansprüche

1. Zusammensetzung zum Waschen und zum Konditionieren von Keratinsubstanzen, insbesondere der Haare und der Haut, **dadurch gekennzeichnet, daß** sie in einem kosmetisch akzeptablen wäßrigen Medium mindestens ein Silicon, mindestens einen grenzflächenaktiven Stoff, der reinigende Eigenschaften hat, und mindestens ein Galactomannan enthält, welches hydrophobe Substituenten aufweist, wobei die hydrophoben Substituenten unter den geradkettigen oder verzweigten Alkyl- und Alkenylgruppen mit 8 bis 60 Kohlenstoffatomen und vorzugsweise 10 bis 32 Kohlenstoffatomen und deren Gemischen ausgewählt sind, wobei die Alkyl- oder Alkenylgruppen mit einer oder mehreren Hydroxygruppen substituiert sein können.

2. Zusammensetzung nach Anspruch 1, **dadurch gekennzeichnet, daß** das Galactomannan hydrophile Substituenten und hydrophobe Substituenten aufweist.

3. Zusammensetzung nach Anspruch 1 oder 2, **dadurch gekennzeichnet, daß** das Galactomannan insgesamt eine molare Substitution über 0,7 aufweist.

4. Zusammensetzung nach Anspruch 2 oder 3, **dadurch gekennzeichnet, daß** das Galactomannan 0,7 bis 4 hydrophile Substituenten und 0,0001 bis 0,02 hydrophobe Substituenten pro Anhydroglykosideinheit aufweist.

5. Zusammensetzung nach einem der Ansprüche 2 bis 4, **dadurch gekennzeichnet, daß** das Molverhältnis von hydrophilen Substituenten und hydrophoben Substituenten im Bereich von 35:1 bis 40 000:1 liegt.

6. Zusammensetzung nach einem der Ansprüche 2 bis 5, **dadurch gekennzeichnet, daß** die hydrophilen Substituenten unter den C₁₋₄-Alkylgruppen, Hydroxymethyl, Hydroxyethyl, Hydroxypropyl, Hydroxybutyl, Carboxymethyl und den aminierten Gruppen und den Carboxygruppen ausgewählt sind.

7. Zusammensetzung nach einem der Ansprüche 1 bis 6, **dadurch gekennzeichnet, daß** das Galactomannan ein Guargummi ist.

8. Zusammensetzung nach einem der Ansprüche 1 bis 7, **dadurch gekennzeichnet, daß** die Silicone unter den in der Zusammensetzung unlöslichen Polyorganosiloxanen ausgewählt sind und in Form von Ölen, Wachsen, Harzen oder Gummis vorliegen.

9. Zusammensetzung nach einem der Ansprüche 1 bis 8, **dadurch gekennzeichnet, daß** die Polyorganosiloxane nicht flüchtige Polyorganosiloxane sind, die unter den Polyalkylsiloxanen, Polyarylsiloxanen, Polyalkylarylsiloxanen, Silicongummis, Siliconharzen, mit organofunktionellen Gruppen modifizierten Polyorganosiloxanen sowie deren Gemischen ausgewählt sind.

10. Zusammensetzung nach einem der Ansprüche 1 bis 9, **dadurch gekennzeichnet, daß**
(a) die Polyalkylsiloxane ausgewählt sind unter:
- den Polydimethylsiloxanen mit Trimethylsilylendgruppen;
- den Polydimethylsiloxanen mit Dimethylsilanolendgruppen;
- den Poly(C₁₋₂₀)alkylsiloxanen;
(b) die Polyalkylarylsiloxane ausgewählt sind unter:
- den Polydimethylmethylphenylsiloxanen, den geradkettigen und/oder verzweigten Polydimethyldiphenylsiloxanen mit einer Viskosität bei 25 °C von 1·10⁻⁵ bis 5·10⁻² m²/s;
(c) die Silicongummis unter den Polydiorganosiloxanen ausgewählt sind, die ein Gewichtsmittel des Molekulargewichts von 200 000 bis 1 000 000 aufweisen und die alleine oder in Form eines Gemisches in einem Lösungsmittel eingesetzt werden;
(d) -die Harze unter, den Harzen ausgewählt sind, die aus den folgenden Einheiten bestehen:
R₃ Si O_{1/2}, R₂ Si O_{2/2}, R Si O_{3/2}, Si O_{4/2},
wobei R eine Kohlenwasserstoffgruppe mit 1 bis 16 Kohlenstoffatomen oder Phenyl bedeutet;
(e) die organomodifizierten Silicone unter den Siliconen ausgewählt sind, die in ihrer Struktur eine oder mehrere organofunktionelle Gruppen aufweisen, die über eine Kohlenwasserstoffgruppe gebunden sind.

11. Zusammensetzung nach Anspruch 10, **dadurch gekennzeichnet, daß** die Silicongummis, die einzeln oder in Form von Gemischen verwendet werden, unter den folgenden Strukturen ausgewählt sind:
- Polydimethylsiloxan
- Polydimethylsiloxan/Methylvinylsiloxanen
- Polydimethylsiloxan/Diphenylsiloxan
- Polydimethylsiloxan/Phenylmethylsiloxan
- Polydimethylsiloxan/Diphenylsiloxan/Methylvinylsiloxanen und den folgenden Gemischen:
- Gemischen, die aus einem am Kettenende hydroxylierten Polydimethylsiloxan und einem cyclischen Polydimethylsiloxan gebildet sind;
- Gemischen, die aus einem Polydimethylsiloxangummi und einem cyclischen Siloxan gebildet sind; und
- Gemischen von Polydimethylsiloxanen unterschiedlicher Viskositäten.

12. Zusammensetzung nach Anspruch 10, **dadurch gekennzeichnet, daß** die organomodifizierten Silicone unter den Polyorganosiloxanen ausgewählt sind, die enthalten:
a) Polyethylenoxid- und/oder Polypropylenoxidgruppen;
b) substituierte oder unsubstituierte aminierte Gruppen;
c) Thiolgruppen;
d) Alkoxygruppen;
e) Hydroxyalkylgruppen, wobei sie der folgenden Formel entsprechen: 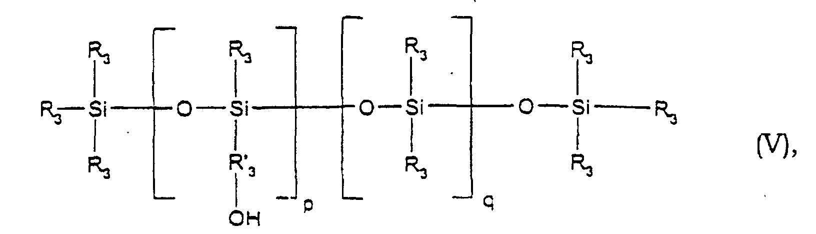 worin die Gruppen R₃, die identisch oder voneinander verschieden sind, unter Methyl und Phenyl ausgewählt sind, wobei mindestens 60 Mol-% der Gruppen R₃ Methyl bedeuten; die Gruppe R'₃ eine zweiwertige Alkylengruppe mit 2 bis 18 Kohlenstoffatomen ist; p im Bereich von 1 bis 30 liegt; und q im Bereich von 1 bis 150 liegt;
f) Acyloxyalkylgruppen, wobei sie der folgenden Formel entsprechen: 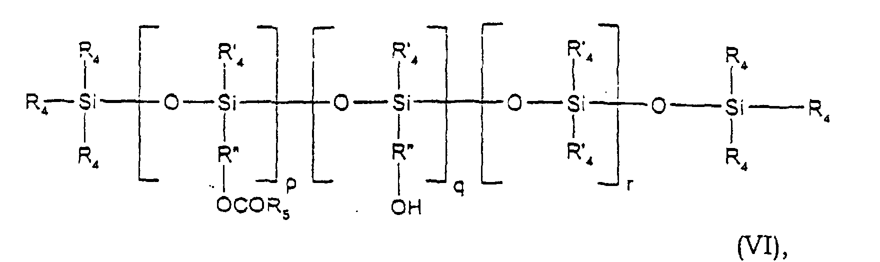 worin bedeuten:
R₄ Methyl, Phenyl, -OCOR₅, Hydroxy, wobei nur eine Gruppe
R₄ pro Siliciumatom OH bedeuten kann;
R'₄ Methyl, Phenyl; wobei mindestens 60 Mol-% der gesamten Gruppen R₄ und R'₄ Methyl bedeuten;
R₅ Alkyl oder C₈₋₂₀-Alkenyl;
R" eine zweiwertige, geradkettige oder verzweigte Alkylengruppe mit 2 bis 18 Kohlenstoffatomen; und worin
r im Bereich von 1 bis 120 liegt;
p im Bereich von 1 bis 30 liegt;
q 0 ist oder unter 0,5 p liegt, wobei p + q im Bereich von 1 bis 30 liegt; und wobei die Polyorganosiloxane der Formel (VI) die folgenden Gruppen 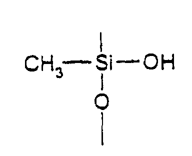
in Mengenanteilen enthalten können, die 15 % der Summe p + q + r nicht übersteigen;
d) Alkylcarboxygruppen,
h) 2-Hydroxyalkylsulfonatgruppen,
i) 2-Hydroxyalkylthiosulfonatgruppen, und
j) Hydroxyacylaminogruppen.

13. Zusammensetzung nach einem der Ansprüche 1 bis 12, **dadurch gekennzeichnet, daß** die Polyorganosiloxane unter den Polyalkylsiloxanen mit Trimethylsilylendgruppen, den Polyalkylsiloxanen mit Dimethylsilanolendgruppen, den Polyalkylarylsiloxanen, den Gemischen von zwei PDMS, die aus einem Gummi und einem Öl mit unterschiedlichen Viskositäten bestehen, den Gemischen von Organosiloxanen und cyclischen Siliconen, den Organopolysiloxanharzen und den Polysiloxanen mit aminierten Gruppen ausgewählt sind.

14. Zusammensetzung nach einem der Ansprüche 1 bis 8, **dadurch gekennzeichnet, daß** die Silicone unter den flüchtigen Siliconen ausgewählt sind.

15. Zusammensetzung nach einem der Ansprüche 1 bis 14, **dadurch gekennzeichnet, daß** die reinigenden grenzflächenaktiven Stoffe unter den anionischen, amphoteren oder nichtionischen grenzflächenaktiven Stoffen oder deren Gemischen ausgewählt sind.

16. Zusammensetzung nach einem der Ansprüche 1 bis 15, **dadurch gekennzeichnet, daß** Gemische von grenzflächenaktiven Stoffen verwendet werden, die unter den Gemischen von anionischen grenzflächenaktiven Stoffen und amphoteren oder nichtionischen grenzflächenaktiven Stoffen ausgewählt sind.

17. Zusammensetzung nach einem der Ansprüche 1 bis 16, **dadurch gekennzeichnet, daß** das Silicon oder die Silicone in den erfindungsgemäßen Zusammensetzungen in Mengenanteilen von 0,05 bis 20 % und vorzugsweise 0,1 bis 10 Gew.-%, bezogen auf das Gesamtgewicht der Zusammensetzung, verwendet werden.

18. Zusammensetzung nach einem der Ansprüche 1 bis 17, **dadurch gekennzeichnet, daß** die hydrophoben Galactomannane in Mengenanteilen von 0,1 bis 10 Gew.-%, bezogen auf das Gesamtgewicht der Zusammensetzung, und insbesondere 0,2 bis 5 % vorliegen.

19. Zusammensetzung nach einem der Ansprüche 1 bis 18, **dadurch gekennzeichnet, daß** die grenzflächenaktiven Stoffe in Mengenanteilen vorliegen, die ausreichend sind, um der Zusammensetzung einen reinigenden Charakter zu geben.

20. Zusammensetzung nach Anspruch 19, **dadurch gekennzeichnet, daß** die grenzflächenaktiven Stoffe in Mengenanteilen von 5 bis 50 Gew.-%, bezogen auf das Gesamtgewicht der Zusammensetzung, und insbesondere 8 bis 35 Gew.-% vorliegen.

21. Zusammensetzung nach einem der Ansprüche 1 bis 20, **dadurch gekennzeichnet, daß** das wäßrige Medium aus Wasser oder einem Gemisch von Wasser und einem kosmetisch akzeptablen Lösungsmittel besteht, das unter den niederen Alkoholen, Alkylenglykolen und Glykolethern ausgewählt ist.

22. Zusammensetzung nach einem der Ansprüche 1 bis 21, **dadurch gekennzeichnet, daß** sie ferner ein kationisches Polymer enthält.

23. Verwendung der Zusammensetzung nach einem der Ansprüche 1 bis 22 als Haarwaschmittel.

24. Verwendung der Zusammensetzung nach einem der Ansprüche 1 bis 22 als Duschgel.

25. Verfahren zum Waschen und Konditionieren von Keratinsubstanzen, **dadurch gekennzeichnet, daß** auf die Keratinsubstanzen mindestens eine Zusammensetzung nach einem der Ansprüche 1 bis 22 aufgetragen wird und die behandelten Keratinsubstanzen gegebenenfalls nach einer Einwirkzeit mit Wasser gespült werden.

26. Verwendung eines hydrophoben Galactomannans nach einem der Ansprüche 1 bis 7 als Mittel zur Suspendierung eines Silicons in einer Zusammensetzung zum Waschen und Konditionieren, die in einem kosmetisch akzeptablen Medium grenzflächenaktive Stoffe enthält.

## Claims

1. Composition for washing and conditioning keratin substances, in particular the hair and the skin, **characterized in that** it comprises, in a cosmetically acceptable aqueous medium, at least one silicone, at least one surfactant with detergent properties and at least one galactomannan gum which contains hydrophobic substituents, the hydrophobic substituents being chosen from linear or branched alkyls and alkenyls containing from 8 to 60 carbon atoms and preferably from 10 to 32 carbon atoms, and mixtures thereof, and the alkyl or alkenyl groups can be substituted with one or more hydroxyls.

2. Composition according to Claim 1, **characterized in that** the galactomannan gum contains hydrophilic substituents and hydrophobic substituents.

3. Composition according to Claim 1 or 2, **characterized in that** the galactomannan gum has a total molar substitution of greater than 0.7.

4. Composition according to Claim 2 or 3, **characterized in that** the galactomannan gum comprises from 0.7 to 4 hydrophilic substituents and from 0.0001 to 0.02 hydrophobic substituents per anhydroglucoside unit.

5. Composition according to any one of Claims 2 to 4, **characterized in that** the molar ratio of the hydrophilic substituents/hydrophobic substituents is between 35:1 and 40,000:1.

6. Composition according to any one of Claims 2 to 5, **characterized in that** the hydrophilic substituents are chosen from C₁-C₄ alkyl groups, hydroxymethyl, hydroxyethyl, hydroxypropyl, hydroxybutyl and carboxymethyl groups and amino and carboxylic groups.

7. Composition according to any one of Claims 1 to 6, **characterized in that** the galactomannan gum is a guar gum.

8. Composition according to any one of Claims 1 to 7, **characterized in that** the silicones are chosen from polyorganosiloxanes that are insoluble in the composition and are in the form of oils, waxes, resins or gums.

9. Composition according to any one of Claims 1 to 8, **characterized in that** the polyorganosiloxanes are non-volatile polyorganosiloxanes chosen from polyalkylsiloxanes, polyarylsiloxanes, polyalkylarylsiloxanes, silicone gums and resins and polyorganosiloxanes modified with organofunctional groups, and mixtures thereof.

10. Composition according to any one of Claims 1 to 9, **characterized in that**:
(a) the polyalkylsiloxanes are chosen from:
- polydimethylsiloxanes containing trimethylsilyl end groups;
- polydimethylsiloxanes containing dimethylsilanol end groups;
- poly(C₁-C₂₀)alkylsiloxanes;
(b) the polyalkylarylsiloxanes are chosen from:
- linear and/or branched polydimethylmethylphenylsiloxanes and polydimethyldiphenylsiloxanes with a viscosity of between 1 x 10⁻⁵ and 5 x 10⁻² m²/s at 25°C;
(c) the silicone gums are chosen from polydiorganosiloxanes with weight-average molecular masses of between 200,000 and 1,000,000, used alone or in the form of a mixture in a solvent;
(d) the resins are chosen from resins consisting of units:
R₃SiO_{1/2}, R₂SiO_{2/2}, RSiO_{3/2}, SiO_{4/2}
in which R represents a hydrocarbon-based group containing from 1 to 16 carbon atoms or a phenyl group;
(e) the organomodified silicones are chosen from silicones containing in their structure one or more organofunctional groups attached via a hydrocarbon-based radical.

11. Composition according to Claim 10, **characterized in that** the silicone gums used, alone or in the form of a mixture, are chosen from the following structures:
- polydimethylsiloxane
- polydimethylsiloxane/methylvinylsiloxanes,
- polydimethylsiloxane/diphenylsiloxane,
- polydimethylsiloxane/phenylmethylsiloxane,
- polydimethylsiloxane/diphenylsiloxane/ methylvinylsiloxanes and the following mixtures:
- mixtures formed from a polydimethylsiloxane hydroxylated at the end of the chain and from a cyclic polydimethylsiloxane;
- mixtures formed from a polydimethylsiloxane gum and from a cyclic silicone; and
- mixtures of polydimethylsiloxanes of different viscosities.

12. Composition according to Claim 10, **characterized in that** the organomodified silicones are chosen from polyorganosiloxanes containing:
a) polyethylenoxy and/or polypropylenoxy groups;
b) substituted or unsubstituted amine groups;
c) thiol groups;
d) alkoxylated groups;
e) hydroxyalkyl groups corresponding to the following formula: 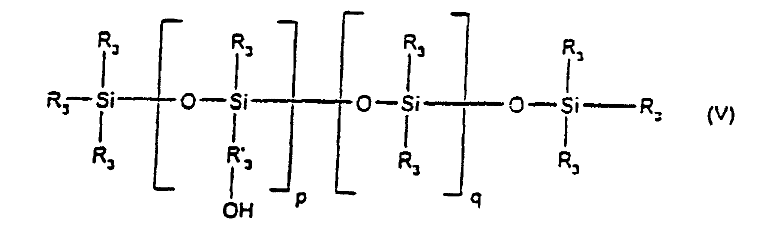 in which the radicals R₃, which may be identical or different, are chosen from methyl and phenyl radicals, at least 60 mol% of the radicals R₃ denoting methyl; the radical R'₃ is a divalent C₂-C₁₈ hydrocarbon-based alkylene chain unit; p is between 1 and 30 inclusive; q is between 1 and 150 inclusive;
f) acyloxyalkyl groups corresponding to the following formula: 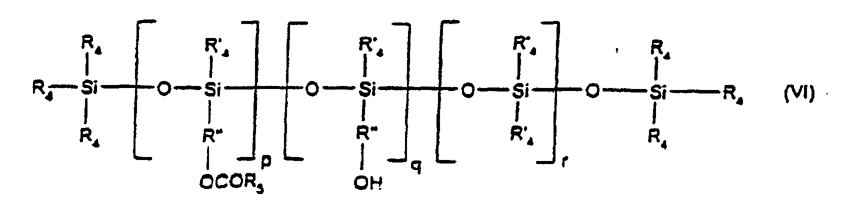 in which:
R₄ denotes methyl, phenyl, -OCOR₅ or hydroxyl, only one of which radicals R₄ per silicon atom may be OH;
R'₄ denotes methyl, phenyl; at least 60 mol% of all of the radicals R₄ and R'₄ denoting methyl;
R₅ denotes C₈-C₂₀ alkyl or alkenyl;
R" denotes a linear or branched, divalent C₂-C₁₈ hydrocarbon-based alkylene radical;
r is between 1 and 120 inclusive;
p is between 1 and 30;
q is equal to 0 or is less than 0.5 p, p + q being between 1 and 30; the polyorganosiloxanes of formula (VI) can contain groups: 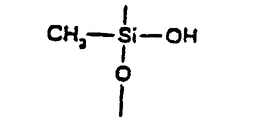 in proportions not exceeding 15% of the sum p + q + r;
g) alkylcarboxylic groups,
h) 2-hydroxyalkyl sulphonate groups,
i) 2-hydroxyalkyl thiosulphonate groups,
j) hydroxyacylamino groups.

13. Composition according to any one of Claims 1 to 12, **characterized in that** the polyorganosiloxanes are chosen from polyalkylsiloxanes containing trimethylsilyl end groups, polyalkylsiloxanes containing dimethylsilanol end groups, polyalkylarylsiloxanes, mixtures of two PDMSs consisting of a gum and an oil of different viscosities, mixtures of organosiloxanes and of cyclic silicones, polyorganosiloxane resins and polysiloxanes with amino groups.

14. Composition according to any one of Claims 1 to 8, **characterized in that** the silicones are chosen from volatile silicones.

15. Composition according to any one of Claims 1 to 14, **characterized in that** the detergent surfactants are chosen from anionic, amphoteric and nonionic surfactants, or mixtures thereof.

16. Composition according to any one of Claims 1 to 15, **characterized in that** mixtures of surfactants chosen from mixtures of anionic surfactants with amphoteric or nonionic surfactants are used.

17. Composition according to any one of Claims 1 to 16, **characterized in that** the silicone(s) is (are) used in the compositions in accordance with the invention in proportions of between 0.05 and 20%, and preferably between 0.1 and 10%, by weight, relative to the total weight of the composition.

18. Composition according to any one of Claims 1 to 17, **characterized in that** the hydrophobic galactomannan gums are present in proportions of between 0.1 and 10% by weight relative to the total weight of the composition, and in particular between 0.2 and 5%.

19. Composition according to any one of Claims 1 to 18, **characterized in that** the surfactants are present in a sufficient proportion to give the composition a detergent nature.

20. Composition according to Claim 19, **characterized in that** the surfactants are present in proportions of between 5 and 50% by weight relative to the total weight of the composition, in particular between 8 and 35% by weight.

21. Composition according to any one of Claims 1 to 20, **characterized in that** the aqueous medium consists of water or of a mixture of water and a cosmetically acceptable solvent chosen from lower alcohols, alkylene glycols and glycol ethers.

22. Composition according to any one of Claims 1 to 21, **characterized in that** it also comprises a cationic polymer.

23. Use, as a shampoo, of the composition as defined in any one of Claims 1 to 22.

24. Use, as a shower gel, of the composition as defined in any one of Claims 1 to 22.

25. Process for washing and conditioning keratin substances, **characterized in that** at least one composition as defined in any one of Claims 1 to 22 is applied to these substances and, after an optional period of leaving the composition to stand on the substances, the treated substances are rinsed with water.

26. Use of a hydrophobic galactomannan gum as defined in any one of Claims 1 to 7, as an agent for suspending a silicone in a washing and conditioning composition containing surfactants in a cosmetically acceptable aqueous medium.
